# EUROPÄISCHE PATENTSCHRIFT

(11) **EP 2 814 577 B1**
(45) Veröffentlichungstag und Bekanntmachung des Hinweises auf die Patenterteilung: **26.05.2021**
(21) Anmeldenummer: 13705138.9
(22) Anmeldetag: 12.02.2013
(51) Int. Cl.: A61Q 19/00, A61K 8/37, A61K 8/06

(54) **STABILE WASSER IN ÖL EMULSIONEN MIT VERBESSERTER SENSORIK**
STABLE WATER-IN-OIL EMULSIONS WITH IMPROVED SENSORY PROPERTIES
ÉMULSIONS EAU DANS L'HUILE STABLES AMÉLIORÉES EN TERME DE PERCEPTION SENSORIELLE

(30) Priorität: 16.02.2012 DE 102012202337
(43) Veröffentlichungstag der Anmeldung: 24.12.2014
(73) Patentinhaber: Beiersdorf AG, 20253 Hamburg (DE)
(72) Erfinder: KOCH, Petra, 22457 Hamburg (DE); BALCKE, Isabel, 22337 Hamburg (DE); MÖLLGAARD, Svenja, Lena, 22337 Hamburg (DE); VON DER FECHT, Stephanie, 22880 Wedel (DE)
(74) Vertreter: Hartmann, Jost
(86) Internationale Anmeldenummer: PCT/EP2013/052735
(87) Internationale Veröffentlichungsnummer: WO 2013/120825

(56) Entgegenhaltungen:
- EP-A1- 1 557 153
- EP-A2- 1 683 781
- WO-A1-2005/117825
- DE-A1- 10 107 628
- DE-C1- 19 641 604
- DATABASE GNPD [Online] MINTEL; 31. August 2001 (2001-08-31), Uroda: "Kwiaty Polskie", XP002716733, Database accession no. 112458
- MEYER J ET AL: "A novel PEG-free emulsifier designed for formulating W/O lotions with a light skin feel", SOFW-JOURNAL SEIFEN, OELE, FETTE, WACHSE, VERLAG FUR CHEMISCHE INDUSTRIE, AUGSBURG, DE, Bd. 131, Nr. 11, 1. Januar 2005 (2005-01-01), Seiten 20-28, XP009104229, ISSN: 0942-7694

## Beschreibung

Die Erfindung ist in den Ansprüchen definiert und umfasst kosmetische und/oder dermatologische Zubereitung auf Basis einer Wasser in Öl Emulsion umfassend als W/O-Emulgatoren Diisostearoyl Polyglyceryl-3 Dimer Dilinoleat und Polyglyceryl-3 Diisostearat.

Als kosmetische oder medizinische Zubereitungen sind oftmals Emulsionen, insbesondere W/O-, O/W-, O/W/O oder W/O/W-Emulsionen, im Einsatz. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten (W/O) in Form feinster Tröpfchen in der anderen Flüssigkeit dispergiert. Die Flüssigkeiten (rein oder als Lösungen) liegen in einer Emulsion in einer mehr oder weniger feinen Verteilung vor, die im Allgemeinen nur begrenzt stabil ist.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter, zum Beispiel elektrische Leitfähigkeit, Sensorik, Anfärbbarkeit der kontinuierlichen Phase, einer O/W-Emulsion ist durch das Wasser geprägt. Bei einer Wasser-in-ÖI-Emulsion (W/O-Emulsion, z. B. Butter) handelt es sich um das umgekehrte Prinzip, wobei der Grundcharakter hier durch das Öl bestimmt wird.

Der Stand der Technik kennt mehrere wesentliche Faktoren, die einen positiven Einfluss auf die Stabilität und Rheologie von Emulsionen haben.

Emulsionen benötigen zu ihrer Bildung und zur Stabilisierung im Allgemeinen einen oder mehrere Emulgatoren, Verdicker und/oder Konsistenzgeber, um über einen kosmetisch akzeptablen Zeitraum stabil zu sein, im Allgemeinen 1 Jahr nach dem Öffnen einer kosmetischen Zubereitung.

Eine besondere Herausforderung stellt die Formulierung von fließfähigen Emulsionen dar. Diese werden aufgrund ihrer ansprechenden Verteilbarkeit vom Verbraucher sehr geschätzt, jedoch ist die stabile Formulierung eine technologische Herausforderung.

Zur Stabilisierung von Emulsionen werden häufig ethoxilierte Emulgatoren verwendet, die, allgemein bekannt, zu belastbaren, stabilen Emulsionszubereitungen führen und oft einen relativ breiten sensorischen Bereich abdecken können. Bekannt ist jedoch, dass ethoxilierte Emulgatoren durch ihre PEG-Einheiten als Penetrationsenhancer wirken.

So offenbart EP 1 192 935 A2 W/O-Emulsionen umfassend Polyether wie PEG-45 (dodecyl glycol) copolymer und PEG-22 (dodecyl glycol) copolymere.

Die Verwendung von Polyethylenglykolen und/oder Polyethylenglykolderivaten und deren Abkömmlingen werden in der Öffentlichkeit kontrovers diskutiert, da sie im Verdacht stehen nach topische Applikation die Haut durchlässiger für Fremdkörper wie z.B. Schadstoffe zu machen.
Weiterhin können unter Einwirkung von Sonnenstrahlung die photoinstabilen Polyethylenglykolhaltigen (PEG)-Emulgatoren zersetzt werden und unschöne Hautreaktionen auslösen.
Aus den genannten Gründen werden vom Verbraucher zunehmend kosmetische Formulierungen gesucht, die frei von dieser Substanzklasse sind.
Auch um W/O-Emulsionen sensorisch ansprechend entwickeln zu können, werden derzeit meist PEG-Emulgatoren verwendet. Da viele Verbraucher Produkte suchen, die frei von dieser Substanz-Klasse sind, ist es eine technologische Herausforderung W/O-Emulsionen ohne diese Stoffe zu stabilisieren und gleichzeitig ein ansprechendes Hautgefühl zu erlangen. Hinsichtlich der Stabilität ist die Herausforderung insbesondere das scaling-up und beim Hautgefühl sind Parameter wie "schnelles und leichtes Einziehen, geringe Klebrigkeit" schwer ohne PEG-Stabilisatoren zu erreichen

Wünschenswert ist es daher Emulsionszubereitung ohne ethoxilierte Emulgatoren zur Verfügung zu stellen, die aber dennoch möglichst breit variierbar und vor allem stabile Emulsionen darstellen.
Weiterhin müssen kosmetische oder dermatologische Zubereitungen einigen ästhetischen und sensorischen Gesichtspunkten genügen um eine ausreichende Verbraucherakzeptanz zu erlangen.

Im Markt ist eine kosmetische Zubereitung "Bebe Zartpflege Zartcreme" mit den Inhaltsstoffen Methylglucoseisostearat, hydrogenated castor oil und Diisotearyl polyglyceryl-3 Dimer Dilinoleat.
Der Zusatz an Hautbefeuchtungsmitteln führt häufig dazu, dass die Zubereitungen klebrig wirken.
DE 60 2004 013 358 T2 offenbart mehrphasige Emulsionen. Bei den beschriebenen Emulsionen handelt es sich um "Mehrfach-Wasser-in-Öl-in-Wasser-Emulsionen" d.h. W/O/W-Systeme. Darüber hinaus wird als W/O-Emulgator Polyglyceryl-3 Diisostearat genannt.

DE 10 2008 028 822 A1 offenbart ein umfangreiches Naschlagewerk zu Emulgatoren und Ölen. Konkret werden kosmetische Stiftzusammensetzungen in Form einer Öl in Wasser Dispersion/Emulsion beschrieben.

DE 199 24 277 A1 beschreibt W/O-Emulsionen und ihre bekannten Vor- und Nachteile. Die Nachteile sollen durch den Einsatz von grenzflächenaktiven Substanzen aus der Gruppe der Alkylmethiconcoplyole beseitigt werden.

In der DE 60100140 T2 werden W/O-Emulsionen mit ihren bekannten Vor- und Nachteilen beschrieben, wobei die Kompensation der Nachteile durch den Einsatz eines speziellen Emulgators erfolgen soll. Polyglyceryl-3 Diisostearate wird hierbei nicht als essentieller Bestandteil genannt. Diisostearoyl Polyglyceryl-3 Dimer Dilinoleat wird nicht genannt.

Weiterhin müssen daher kosmetische oder dermatologische Zubereitungen einigen ästhetischen und sensorischen Gesichtspunkten genügen um eine ausreichende Verbraucherakzeptanz zu erlangen.
Für W/O-Emulsionen ist bekannt, dass die Viskosität abhängig von der eingesetzten Emulgatormenge ist. Wird eine hohe Konzentration eingesetzt, werden Cremes mit einer hohen Konsistenz erhalten, bei Reduktion der Einsatzkonzentration sind die Systeme zwar fließfähig, oft aber nicht mehr stabil, dies zeigt sich insbesondere im up-sacling. Somit stellt die Formulierung von fließfähigen W/O-Emulsionen derzeit eine technologische Herausforderung dar.
Eine für den Verbraucher sehr wesentliche, dabei aber nur schwer quantitativ messbare Eigenschaft kosmetischer Produkte ist ihre Textur und Sensorik. Unter dem Begriff "Textur" werden diejenigen Eigenschaften eines Kosmetikums verstanden, die auf den Gefügebau der Zubereitung zurückgehen, durch Tast- und Berührungssinne empfunden und ggf. in mechanischen oder rheologischen Fließeigenschaften ausgedrückt werden können. Die Textur kann insbesondere mittels Sensorik getestet werden. Die gegebenenfalls mit Hilfe von Zusatzstoffen beeinflussbare Textur kosmetischer Produkte ist für den Verbraucher von nahezu gleicher Bedeutung wie deren objektiv feststellbaren Wirkungen.

Mit dem Begriff "Sensorik" wird die wissenschaftliche Disziplin bezeichnet, die sich mit der Bewertung von kosmetischen Zubereitungen auf Grund von Sinneseindrücken befasst. Die sensorische Beurteilung eines Kosmetikums erfolgt anhand der visuellen, olfaktorischen und haptischen Eindrücke.
- Visuelle Eindrücke: alle mit dem Auge wahrnehmbaren Merkmale (Farbe, Form, Struktur).
- Olfaktorische Eindrücke: alle beim Einziehen von Luft durch die Nase wahrnehmbaren Geruchseindrücke, die häufig in Anfangsgeruch (Kopfnote), Hauptgeruch (Mittelnote, Körper) und Nachgeruch (Ausklang) differenziert werden können. Auch die erst bei der Anwendung freigesetzten flüchtigen Stoffe tragen zum olfaktorischen Eindruck bei.
- Haptische Eindrücke: alle Empfindungen des Tastsinns, die vornehmlich Gefüge und Konsistenz des Produktes betreffen.

Die sensorische Analyse macht von der Möglichkeit Gebrauch, den sensorischen Gesamteindruck eines Produktes integral zu erfassen. Nachteile der sensorische Analyse sind die Subjektivität des Eindrucks, eine leichte Beeinflussbarkeit der Prüfpersonen und die dadurch bedingte starke Streuung der Ergebnisse. Diesen Schwächen begegnet man heute durch den Einsatz von Gruppen geschulter Prüfpersonen, gegenseitige Abschirmung der Prüfer sowie statistische Auswertung der meist zahlreichen Analysendaten.

Eine weitere Aufgabe der vorliegenden Erfindung war es daher, Zubereitungen zu Verfügung zu stellen, welche neben den für Kosmetika üblichen Kriterien wie Verträglichkeit, Lagerstabilität und dergleichen auch für den Verbraucher wesentliche, bisher nicht gekannte kosmetische, insbesondere sensorische, Leistungen bieten. Insbesondere war es ein Ziel eine lagerstabile Formulierung zu schaffen, die Pflege vermittelt, ohne dabei glitischig, ölig oder fettig zu sein und gleichzeitig eine eindeutige "Lotion-Anmutung", d.h. fließfähig, und KEINE Creme-Anmutung zeigt.

Die Erfindung ist eine kosmetische und/oder dermatologische Zubereitung auf Basis einer Wasser in Öl Emulsion umfassend als W/O-Emulgatoren Diisostearoyl Polyglyceryl-3 Dimer Dilinoleat und Polyglyceryl-3 Diisostearat, wobei der Anteil an W/O-Emulgatoren im Bereich von 0,1 bis 5 Gew.%, bevorzugt 1 bis 2 Gew.%, besonders bevorzugt 1,3 bis 1,6 Gew.%, bezogen auf die Gesamtmasse der Zubereitung, liegt und wobei ein oder mehrere Puderrohstoffe enthalten sind.

Die Zubereitung umfasst bevorzugt nur diese zwei W/O-Emulgatoren.

Überraschender Weise ist es durch die Kombination dieser zwei erfindungsgemäßen W/O-Emulgatoren gelungen, die genannten technischen Herausforderung zu lösen.

Bevorzugt umfassen die erfindungsgemäßen Zubereitungen keine weiteren zusätzlichen anderen Emulgatoren. Der Anteil an zusätzlichen Emulgatoren sollte somit bevorzugt unter 0,01 Gew.% liegen, bezogen auf die Gesamtmasse der Zubereitung, um als erfindungsgemäß - ohne zusätzlichen Emulgator - zu gelten.

In gleicher Weise kann erfindungsgemäß auf den Zusatz an Polyethylenglykolen und/oder Polyethylenglykolderivaten verzichtet werden. Der Anteil an PEGs liegt daher unter 1 Gew.%, insbesondere 0 Gew.%, bezogen auf die Gesamtmasse der Zubereitung.

Es sind erfindungsgemäß keine weiteren W/O-Emulgatoren zur Stabilisierung notwendig. Hydrophile Stabilisatoren wie Verdicker und Fettalkohole können erfindungsgemäß optional enthalten sein.

Der Anteil an den W/O-Emulgatoren liegt im Bereich von 0,1 bis 5 Gew.%, bevorzugt 1 bis 2 Gew.%, besonders bevorzugt 1,3 bis 1,6 Gew.%, bezogen auf die Gesamtmasse der Zubereitung.

Der Anteil von Polyglyceryl-3 Diisostearate wird dabei bevorzugt gewählt im Bereich von 0,01 bis 2 Gew.%, bevorzugt 0,05 bis 0,5 Gew.%, besonders bevorzugt 0,08 bis 0,2 Gew.%, und an Diisostearoyl Polyglyceryl-3 Dimer Dilinoleate im Bereich 0,09 bis 3 Gew.%, bevorzugt 0,5 bis 2 Gew.%, besonders bevorzugt 1 bis 1,8 Gew.%, bezogen auf die Gesamtmasse der Zubereitung.

Die erfindungsgemäßen Zubereitungen umfassen vorteilhaft mindestens ein Hautbefeuchtungsmittel zu einem Anteil von insgesamt 5 bis 20 Gew.%, bezogen auf die Gesamtmasse der Zubereitung. Bevorzugt ist ein Glycerin-Gehalt von 7 bis 15% beonders bevorzugt von 10%.Insbesondere wird als Hautbefeuchtungsmittel Glycerin gewählt. Durch den Einsatz der erfindungsgemäßen Emulgatorkombination ist es gelungen, eine W/O-Emulsion zu formulieren, die trotz hoher Mengen an Hautbefeuchtungsmitteln wie Glycerin ein sehr ansprechendes Hautgefühl aufweist und bei Streßtests wie z.B. Lagerung bei erhöhten bzw. wechslenden Tempertauren, stabil ist. Diese Stabilität besteht nicht nur bei den im Labormaßstab hergestellten 1 bis 5 kg-Ansätzen sondern auch bei größeren Mengen wie z.B. 100 bis 500 kg und auch bei der Produktion im Tonnen-Maßstab. Dieser als "up-scaling" bekannte Prozess ist bekanntermaßen für übliche W/O-Emulsionen besonders sensibel. Für die im Rahmen dieser Erfindung dargestellten Zubereitungen stellte sich der up-scaling-Prozess als überraschend problemlos dar.

Emulgatoren bewirken, dass zwei nicht miteinander mischbare Flüssigkeiten (zum Beispiel Öl in Wasser) sich zu einer Emulsion vermengen können. Aufgrund des amphiphilen Charakters dringen sie mit ihrem fettlöslichen Teil in das Öl ein. Durch den hydrophilen Teil kann das nun durch Rühren entstandene Öltröpfchen in der wässrigen Umgebung dispergiert werden. Emulgatoren haben primär keinen waschaktiven, tensidischen Charakter. Emulgatoren setzen die Grenzflächenspannung zwischen den beiden Phasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.
Damit Verbindungen als Emulgatoren wirksam sein können, müssen sie eine bestimmte Molekülstruktur aufweisen. Strukturelles Kennzeichen solcher Verbindungen ist ihr amphiphiler Molekülaufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe).
Es wird dabei unterschieden zwischen nicht-ionisch, anionisch und kationischen Emulgatoren. Ein Kennzeichen für die Hydrophilie eines gegebenen Emulgators ist dessen HLB-Wert, der sich nach folgender Formel ergibt: HLB = 20 x (1- Mₗᵢₚₒₚₕᵢₗ/M), wobei Mₗᵢₚₒₚₕᵢₗ für die Molmasse des lipophilen Anteils im Emulgator und M für die Molmasse des gesamten Emulgators steht.

Durch den Einsatz dieser Emulgatorkombination ist es gelungen, eine W/O-Emulsion zu formulieren, die trotz hoher Mengen an Pflegestoffe wie Glycerin ein sehr ansprechendes Hautgefühl aufweist, die fließfähig und auch im upscaling stabil ist.
Bekanntermaßen problematisch ist bei W/O-Formulierungen mit einem hohen Glycerin-Gehalt das Hautgefühl. Überraschend ist, dass durch die Emulgatorkombination aus Polyglyceryl-3 Diisostearate und Diisostearoyl Polyglyceryl-3 Dimer Dilinoleate das Hautgefühl wesentlich verbessert werden konnte. Formulierungen, die beide Emulgatoren enthalten sind deutlich weniger ölig und fettig als Vergleichsformeln, die nur einen der beiden Emulgatoren enthalten.

| Rezeptur | Sensorik |
|---|---|
| Beispiel-Rezeptur 4 | pflegend, angenehm |
| Beispiel-Rezetur ohne Polyglyceryl-3 Diisostearate | Im Vergleich zu Beispiel-Rezeptur 4 fettiger und öliger |

Überraschend war es, dass erfindungsgemäß eine sensorisch gleichwertige bzw. verbesserte Zubereitung entwickelt werden konnte, die PEG-frei ist, mehr als doppelt so viel Glycerin enthält und gut im up-scaling ist.
Die erfindungsgemäßen lagerstabilen Formulierung (s. Beispiele 1 bis 6) vermitteln dem Anwender ein Pflegegefühl ohne dabei glitschig, ölig oder fettig zu sein. Gleichzeitig zeigen die erfindungsgemäßen Zubereitungen eine "Lotion-Anmutung", d.h. sie sind fließfähig und zeigen keine Creme-Anmutung.

Die Erfindung ist eine kosmetische oder dermatologische Zubereitung auf Basis einer Wasser in Öl Emulsion und sie weist insbesondere keine (0%) ethoxilierte Emulgatoren, Polyethylenglykole und/oder Polyethylenglykolderivate auf.

Die erfindungsgemäßen Zubereitungen umfassen vorteilhaft mindestens 10 bis 30 Gew.%, bevorzugt 15 bis 25 Gew.%, besonders bevorzugt 18 bis 22 Gew.% Lipide.
Der Lipid- oder Ölphase werden die Emulgatoren nicht hinzugezählt.

Vorteilhaft kann sich die Ölphase aus bei Raumtemperatur (RT) festen bzw. halbfesten Rohstoffen und flüssigen Rohstoffen zusammen setzen. Der Anteil an festen/bzw. halbfesten Bestandteilen bezogen auf die Ölphase ist ca. 40% bis 0,1%, bevorzugt ca. 30 bis 3 % und besonders bevorzugt ca. 15 bis 5 Gew.%, bezogen auf die Gesamtmasse der Ölphase, gewählt.

Bevorzugte bei RT flüssige Lipidkomponenten werden gewählt aus der Gruppe Paraffinum Liquidum, Isopropyl Palmitate, C13-16 Isoparaffin und natürliche Öle wie Arganöl, Olivenöl, Sonnenblumenöl oder Mandelöl.

Bevorzugt bei Raumtemperatur (RT) feste bzw. halbfeste Lipidkomponenten werden gewählt aus der Gruppe Cera Microcristallina, Cetyl Palmitate und/oder Vaseline (Cera Microcristallina + Paraffinum Liquidum).

Die erfindungsgemäße kosmetische und/oder dermatologische Zubereitung ist eine bei Raumtemperatur fließfähige Wasser in Öl Emulsion und keine Mehrfachemulsion, wie W/O/W- oder O/W/O-Emulsion. Vorteilhaft ist die erfindungsgemäße Emulsion nicht als Mikro- oder Nanoemulsion formuliert.

Die erfindungsgemäßen Zubereitungen sind vorteilhaft bei Raumtemperatur (20°C) fließfähig.

Weiterhin umfassen die erfindungsgemäßen Zubereitungen ein oder mehrere Puderrohstoffe, die bevorzugt zu einem Anteil von bis zu 5 Gew.%, bevorzugt 0,2 bis 2 Gew.%, bezogen auf die Gesamtmasse der Zubereitung enthalten sind. Bevorzugter Puderrohstoff ist Aluminum Starch Octenylsuccinat und/oder Talkum.

Die kosmetischen oder dermatologischen Zubereitungen gemäß der Erfindung können ferner kosmetische Hilfsstoffe und weitere Wirkstoffe enthalten, wie sie üblicherweise in solchen Zubereitungen verwendet werden, z. B. Konservierungsmittel, Konservierungshelfer, Bakterizide, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate, Selbstbräuner, Puffer, pH Regulatoren, Pflanzliche Extrakte, Tenside, Treibgase, Puder, Sebumabsorbierende Substanzen, UV-Filter, Wirkstoffe wie zum Beispiel Anti Age, Anti-Cellulite, Anti Akne, Anti-Rosacea, Anti-Neurodermitis, Antioxidantien, Moisturiser, Chelatbildner, Antitraspirantien, Bleich und Färbemittel etc, sofern der Zusatz die geforderten Eigenschaften hinsichtlich PEG-freiheit, Emulgatorgehalt, geforderter Stabilität und Sensorik nicht behindert.

Der Wassergehalt der erfindungsgemäßen Zubereitungen liegt vorteilhaft zwischen 40 und 80 Gew.%, bevorzugt zwischen 50 Gew.% und 70 Gew.%, besonders bevorzugt zwischen 55 Gew.% und 65 Gew.%, jeweils bezogen auf die Gesamtmasse der Zubereitungen.

Bei Einschränkungen auf bevorzugt genannte Stoffe, seien es die Lipide mit erfindungsgemäßen Spreitwert, die W/O Emulgatoren oder weitere bevorzugt gennannte Bestandteile, so beziehen sich deren bevorzugten Anteilsbereiche dann auch auf die dann ausgewählten Einzelbestandteile. Die anderen, die durch die Einschränkung ausgeschlossenen Bestandteile, zählen dann nicht mehr zu den aufgeführten Anteilsbereichen hinzu.

Die nachfolgenden Beispiele illustrieren die erfindungsgemäßen Zubereitungen. Die Zahlenangaben beziehen sich auf die Gewichtsanteile in Bezug zur Gesamtmasse der Zubereitung, sofern nichts anderes angegeben ist.

### Beispiel 1:

0,1% Polyglyceryl-3 Diisostearate
1,9 % Diisostearoyl Polyglyceryl-3 Dimer Dilinoleate
3% Cera Microcristallina
10% Caprylic/Capric Triglyceride
10% Dicapylyl Ether
1% Talkum
12% Glycerin
1% Propylenglycol
0,1% Hexandiol
0,15% Potassium Sorbate
0,1% Citric Acid
0,2% Sodium Citrate
0,4% Parfüm
Mit Aqua ad 100%

### Beispiel 2:

0,25 % Polyglyceryl-3 Diisostearate
1,9 % Diisostearoyl Polyglyceryl-3 Dimer Dilinoleate
2,5 % Cetylpalmitate
0,1% Cera Microcristallina
4% C13-16 Isoparaffin
4% Argania Spinosa Kernel Oil
6,5% Paraffinum Liquidum
6,5% Isopropyl Palmiate
10% Glycerin
2% Tapoca Starch
0,15% Potassium Sorbate
0,2% Sodium Citrate
0,1% Citric Acid
0,15% Parfüm
Mit Aqua ad 100%

### Beispiel 3:

1,5 % Polyglyceryl-3 Diisostearate
1,5 % Diisostearoyl Polyglyceryl-3 Dimer Dilinoleate
2,7% Shea Butter
7,5% Isohexadecane
5, 3% Dibutyl Adipate
6,5% Paraffinum Liquidum
0,1% Sonnenblumenöl
13,75% Glycerin
0,5% Ethylhexylglycerin
1% Aluminium Starch Octenylsuccinate
1% Nylon-12
0,15% Potassium Sorbate
0,2% Sodium Citrate
0,1% Citric Acid
0,3% Parfüm
Mit Aqua ad 100%

### Beispiel 4:

0,1 % Polyglyceryl-3 Diisostearate
1,4 % Diisostearoyl Polyglyceryl-3 Dimer Dilinoleate
1 % Cera Microcristallina + Paraffinum Liquidum
0,5 % Cera Microcristallina
2 % Paraffinum Liquidum
1% Prunus Amygdalus Dulcis Oil
0,5% Cetyl Palmitate
9,5% Isopropylpalmitate
6% C13-16 Isoparaffin
0,5% Aluminium Starch Octenylsuccinate
10% Glycerin
0,1% Maris Sal
1% Glyceryl Glucose
0,15% Potassium Sorbate
0,2% Sodium Citrate
0,1% Citric Acid
0,35% Parfüm
Mit Aqua ad 100%

### Beispiel 5:

0,3 % Polyglyceryl-3 Diisostearate
0,8 % Diisostearoyl Polyglyceryl-3 Dimer Dilinoleate
0,2 % Cera Microcristallina
1% Shea Butter
2,5% Cera Microcristallina + Paraffinum Liquidum
7,5% Caprylic/Capric Triglyceride
12 % Dicaprylyl Ether
0,1% Olivenöl
1% Talkum
0,2% Aluminium Starch Octenylsuccinate
12% Glycerin
0,1% Caprylyl Glycol
0,15% Potassium Sorbate
0,1% Citric Acid
0,2% Sodium Citrate
0,4% Parfüm
Mit Aqua ad 100%

### Beispiel 6:

0,8 % Polyglyceryl-3 Diisostearate
1,2 % Diisostearoyl Polyglyceryl-3 Dimer Dilinoleate
0,2 % Cera Microcristallina
1% Shea Butter
2,5% Cera Microcristallina + Paraffinum Liquidum
7,5% C13-16 Isoparaffin
12 % Dicaprylyl Ether
0,1% Olivenöl
1% Talkum
0,2% Aluminium Starch Octenylsuccinate
12% Glycerin
0,1% Caprylyl Glycol
0,15% Potassium Sorbate
0,1% Citric Acid
0,2% Sodium Citrate
0,4% Parfüm
Mit Aqua ad 100%

## Patentansprüche

1. Kosmetische und/oder dermatologische Zubereitung auf Basis einer Wasser in Öl Emulsion umfassend als W/O-Emulgatoren Diisostearoyl Polyglyceryl-3 Dimer Dilinoleat und Polyglyceryl-3 Diisostearat,
wobei der Anteil an W/O-Emulgatoren im Bereich von 0,1 bis 5 Gew.%, bevorzugt 1 bis 2 Gew.%, besonders bevorzugt 1,3 bis 1,6 Gew.%, bezogen auf die Gesamtmasse der Zubereitung, liegt und
wobei ein oder mehrere Puderrohstoffe enthalten sind.

2. Zubereitung nach Anspruch 1 **dadurch gekennzeichnet, dass** nur Diisostearoyl Polyglyceryl-3 Dimer Dilinoleat und Polyglyceryl-3 Diisostearat als Emulgatoren enthalten sind.

3. Zubereitung nach einem der vorstehenden Ansprüche umfassend mindestens ein Hautbefeuchtungsmittel zu einem Anteil von insgesamt 5 bis 20 Gew.%, bezogen auf die Gesamtmasse der Zubereitung.

4. Zubereitung nach Anspruch 4 **dadurch gekennzeichnet, dass** als Hautbefeuchtungsmittel Glycerin gewählt wird.

5. Zubereitung nach einem der vorstehenden Ansprüche 2 bis 4 **dadurch gekennzeichnet, dass** der Anteil an Polyglyceryl-3 Diisostearate im Bereich von 0,01 bis 2 Gew.%, bevorzugt 0,05 bis 0,5 Gew.%, besonders bevorzugt 0,08 bis 0,2 Gew.%, und an Diisostearoyl Polyglyceryl-3 Dimer Dilinoleate im Bereich 0,09 bis 3 Gew.%, bevorzugt 0,5 bis 2 Gew.%, besonders bevorzugt 1 bis 1,8 Gew.%, bezogen auf die Gesamtmasse der Zubereitung, gewählt wird.

6. Zubereitung nach Anspruch 1 **dadurch gekennzeichnet, dass** der Anteil an Puderrohstoffen bis zu 5 Gew.%, insbesondere 0,2 bis 1 Gew.%, bezogen auf die Gesamtmasse der Zubereitung, beträgt.

7. Zubereitung nach Anspruch 1 oder 6 **dadurch gekennzeichnet, dass** als Puderrohstoff Aluminum Starch Octenylsuccinat und/oder Talkum gewählt wird.

8. Zubereitung nach einem der vorstehenden Ansprüche **dadurch gekennzeichnet, dass** der Wasseranteil im Bereich von 50 bis 80 Gew.%, insbesondere zwischen 62 und 68 Gew.%, bezogen auf die Gesamtmasse der Zubereitung, gewählt wird.

9. Zubereitungen nach einem der vorstehenden Ansprüche **dadurch gekennzeichnet, dass** der Anteil an Polyethylenglykolen und/oder Polyethylenglykolderivaten unter 1 Gew.%, insbesondere 0 Gew.%, bezogen auf die Gesamtmasse der Zubereitung, beträgt.

10. Verwendung einer kosmetischen und/oder dermatologischen Zubereitung gemäß Anspruch 1 zur Verbesserung der sensorischen Eigenschaften im Vergleich zu einer Zubereitung enthaltend nur den W/O-Emulator Polyglyceryl -3 Dimer Dilinoleat.

## Claims

1. Cosmetic and/or dermatological preparation based on a water in oil emulsion comprising diisostearoyl polyglyceryl-3 dimer dilinoleate and polyglyceryl-3 diisostearate as W/O emulsifiers,
wherein the fraction of W/O emulsifiers is in the range of 0.1 to 5% by weight, preferably 1 to 2% by weight, particularly preferably 1.3 to 1.6% by weight, based on the total mass of the preparation, and
wherein one or more powder raw materials are present.

2. Preparation according to Claim 1, **characterized in that** the only emulsifiers present are diisostearoyl polyglyceryl-3 dimer dilinoleate and polyglyceryl-3 diisostearate.

3. Preparation according to either of the preceding claims comprising at least one skin moisturizer at a proportion of in total 5 to 20% by weight, based on the total mass of the preparation.

4. Preparation according to Claim 4, **characterized in that** the skin moisturizer selected is glycerin.

5. Preparation according to any of preceding Claims 2 to 4, **characterized in that** the fraction of polyglyceryl-3 diisostearate is selected in the range of 0.01 to 2% by weight, preferably 0.05 to 0.5% by weight, particularly preferably 0.08 to 0.2% by weight, and that of diisostearoyl polyglyceryl-3 dimer dilinoleate is selected in the range of 0.09 to 3% by weight, preferably 0.5 to 2% by weight, particularly preferably 1 to 1.8% by weight, based on the total mass of the preparation.

6. Preparation according to Claim 1, **characterized in that** the fraction of powder raw materials is up to 5% by weight, especially 0.2 to 1% by weight, based on the total mass of the preparation.

7. Preparation according to Claims 1 or 6, **characterized in that** the powder raw material selected is aluminium starch octenylsuccinate and/or talc.

8. Preparation according to any of the preceding claims, **characterized in that** the proportion of water is selected in the range of 50 to 80% by weight, particularly between 62 and 68% by weight, based on the total mass of the preparation.

9. Preparations according to any of the preceding claims, **characterized in that** the fraction of polyethylene glycols and/or polyethylene glycol derivatives is below 1% by weight, especially 0% by weight, based on the total mass of the preparation.

10. Use of a cosmetic and/or dermatological preparation according to Claim 1 for improving the sensory properties compared to a preparation only comprising the W/O emulsifier polyglyceryl-3 dimer dilinoleate.

## Revendications

1. Préparation cosmétique et/ou dermatologique à base d'une émulsion eau dans huile comprenant en tant qu'émulsifiants E/H un dilinoléate de polyglycéryl-3 dimère de diisostéaroyle et un diisostéarate de polyglycéryl-3,
la proportion en émulsifiants E/H se situant dans la plage de 0,1 à 5 % en poids, préférablement de 1 à 2 % en poids, particulièrement préférablement de 1,3 à 1,6 % en poids, par rapport à la masse totale de la préparation, et
une ou plusieurs matières premières de poudre étant contenues.

2. Préparation selon la revendication 1 **caractérisée en ce qu'**uniquement du dilinoléate de polyglycéryl-3 dimère de diisostéaroyle et du diisostéarate de polyglycéryl-3 sont contenus en tant qu'émulsifiants.

3. Préparation selon l'une quelconque des revendications précédentes comprenant au moins un agent d'hydratation de la peau en une proportion totale de 5 à 20 % en poids, par rapport à la masse totale de la préparation.

4. Préparation selon la revendication 4, **caractérisée en ce que** de la glycérine est choisie en tant qu'agent d'hydratation de la peau.

5. Préparation selon l'une quelconque des revendications précédentes 2 à 4, **caractérisée en ce que** la proportion de diisostéarates de polyglycéryl-3 est choisie dans la plage de 0,01 à 2 % en poids, préférablement de 0,05 à 0,5 % en poids, particulièrement préférablement de 0,08 à 0,2 % en poids, et la proportion de dilinoléates de polyglycéryl-3 dimère de diisostéaroyle est choisie dans la plage de 0,09 à 3 % en poids, préférablement 0,5 à 2 % en poids, particulièrement préférablement 1 à 1,8 % en poids, par rapport à la masse totale de la préparation.

6. Préparation selon la revendication 1, **caractérisée en ce que** la proportion en matières premières de poudre va jusqu'à 5 % en poids, en particulier de 0,2 à 1 % en poids, par rapport à la masse totale de la préparation.

7. Préparation selon la revendication 1 ou 6, **caractérisée en ce qu'**en tant que matière première de poudre, de l'octénylsuccinate d'amidon d'aluminium et/ou du talc est choisi.

8. Préparation selon l'une quelconque des revendications précédentes, **caractérisée en ce que** la proportion d'eau est choisie dans la plage de 50 à 80 % en poids, en particulier entre 62 et 68 % en poids, par rapport à la masse totale de la préparation.

9. Préparations selon l'une quelconque des revendications précédentes, **caractérisées en ce que** la proportion en polyéthylèneglycol et/ou dérivés de polyéthylèneglycol est inférieure à 1 % en poids, en particulier est de 0 % en poids, par rapport à la masse totale de la préparation.

10. Utilisation d'une préparation cosmétique et/ou dermatologique selon la revendication 1 pour l'amélioration des propriétés sensorielles par rapport à une préparation ne contenant que l'émulsifiant E/H dilinoléate de polyglycéryl-3 dimère.
